# EUROPEAN PATENT APPLICATION

(11) **EP 1 640 750 A1**
(43) Date of publication of application: **29.03.2006**
(21) Application number: 04405408.8
(22) Date of filing: 01.07.2004
(51) Int. Cl.: G02B 5/128, A61B 19/00

(54) **Retro-reflective device and method for forming such retro-reflective devices**

(71) Applicant: Biofluid Systems Société Anonyme, 1260 Nyon (CH)
(72) Inventor: Mosimann, Laurent, 1291 Commugny (CH); Rochat, Jean-Denis, 1272 Genolier (CH)
(74) Representative: Savoye, Jean-Paul

(57) **Abstract**

Retro-reflective device having at least a body (2) comprising salient elements (5b) on an external surface of this body. The body (2) is made of at least one first material entity (7) made of a homogeneous compound of plastic material (4) with on one hand elements (5a) entirely coated with at least one reflective layer (6) and fully embedded inside the plastic material (4), and on the other hand salient elements (5b) partially coated with a same reflective layer, said reflective layer located inside the plastic material (4), and the salient non-coated surface of said elements (5b) protruding out of the surface of the plastic material (4).

## Description

The present invention relates to retro-reflective device, a method for forming such retro-reflective devices and a preferred use of those retro-reflective devices.

Light retro-reflective applications are commonly used in many fields like road marking, road signs, reflective clothes, optical sensors or any other field where a high retro-reflection of light is required. The present invention is designated to manufacture retro-reflective devices of any shape which can find applications in many different fields. More specifically, this invention relates to a tri-dimensional retro-reflective device and to a method to manufacture devices of this kind. By way of non-limitative example, the present invention will refer more particularly to a specific device which is a spherical retro-reflective marker used in Image Guided Surgery systems (IGS). However, it is advisable to note that the present invention does not have any intention to be limited by medical application.

IGS system has been described in the United States Patent 5,817,105 wherein two cameras are used to determine the spatial position and orientation of surgical tools relative to the body of patient to be operated. Each surgical instrument is provided with cabled markers adapted to emit infrared sources allowing to determine the spatial position of the tool by cameras and a computer process. The aim of such a result is to assist the surgeon by displaying images exactly where the surgical instrument is situated during cerebral surgery, for instance.

In order to operate without any cable, the United States Patent 6,351,659 discloses an IGS system using passive reflecting markers instead of prior active signal emitters. Such passive reflectors are made of a plastic sphere, having typically a diameter comprised between 10 and 15 millimeters, covered with a retro-reflective material like a reflecting film. Such a film is glued over the surface of the plastic sphere as described in the description of the prior art of the international publication number WO 01/26574. This technology is manufactured by hand which results in high production costs and a lot of quality control because of delaminating risk, film wrinkling, superposition or uncovering and glue smear. In addition, this technology would be very difficult to automate.

Another solution is given in the United States Patent 2003/0174401 which discloses a passive reflector made of one transparent retro-reflective ball of glass material. This ball can be partially coated with metallization layers so as to achieve a mirror effect. By having such a metallization, the light beams picked up by the cameras can only pass into the ball at a given angle so that the ball needs to be right oriented with respect to the cameras. A variant of this marker consists to have a ball without such a coating. However, the drawback of this solution is that it efficiently reduces the intensity level of the reflected light of the beam.

United States Patent 6,224,219 discloses a retro-reflective film and a method for forming it. This film includes optical elements in the form of transparent micro-spheres that are partially embedded in a binding layer. A reflective coating is disposed between the layer of micro-spheres and the binding layer. The light that enters a micro-sphere can be refracted toward the center of the micro-sphere, reflected off the reflective coating behind the micro-sphere and redirected out of the micro-sphere in the general direction of the incident light. The method refers to a vaporization of a liquid coating material, such as a pre-polymer, through a coating die and onto the optical elements of a retro-reflective web. This web is coated in a continuous fashion by traveling around the surface of a drum. This method makes it only possible to manufacture retro-reflective webs or flat supports and involve condensing of a pre-polymer. Therefore, it is rather designated to perform retro-reflective articles such as road signs, roll up signs or road worker safety vests.

International publication number WO 97/03814 suggests another solution to embedded optical elements such as glass beads into one or several core elements comprising a thermoplastic material. To this end, the technique consists in adding solid core elements to a mobile bed of hot glass beads. Due to the fact that the glass beads were previously heated, they immediately come to attach to a majority of the surface area of the core thermoplastic elements which melt and become tacky on contact with the heated beads of glass. To favor the mixing between core elements and glass beads, the mobile bed of optical elements is jogged for a sufficient time to produce effective optical element embedment in a generally closely packed arrangement. Preferably, the optical elements are embedded on average into the core element to a depth of at least about 50% of their diameter.

One of the drawbacks of this method is that optical elements cannot be partially coated with metallization layers so as to achieve a mirror effect. The random attachment of the micro-spheres achieved by the mobile bed does not allow to obtain a right orientation of the coating portion for all glass beads. Consequently, this method is suitable to manufacture retro-reflective articles with uncoated micro-spheres. Moreover, the embedded depth of the optical elements is rather difficult to control due to the fact that it depends on the temperature of the glass beads relative to the temperature of the core elements, on the amount of beads forming the covering of the core element and also from the duration of the mixing.

The main problem in the above mentioned prior solutions is that the disclosed methods are rather designated to manufacture flat articles such as retro-reflective webs and stiff flat surfaces. On the other hand, these known methods cannot be applied to substantially voluminous articles, in particular tri-dimensional articles of any shapes including concave portions if necessary.

The object of present invention is to remedy at least in part the aforesaid drawbacks. For this purpose, the subject of the present invention is a retro-reflective device according to claim 1, a method for forming retro-reflective devices according to claim 10 and a preferred use of those retro-reflective devices according to claim 16.

The invention will be more clearly understood from the study of the following description of an embodiment, given by way of non-limitative example and schematically illustrated in the enclosed drawings in which :
- Fig. 1a and 1b are partial sectional views of two types of retro-reflective devices, made of a first material entity only, according to the present invention.
- Fig. 2 is a partial sectional view of another retro-reflective device, made of a first material entity and an additional material entity, according to the present invention.
- Fig. 3 is a detailed sectional view of a portion of the spherical retro-reflective device of Fig. 1a.
- Fig. 4a is a schematic illustration of a converting process corresponding to the first stage of the method for forming retro-reflective devices according to the present invention.
- Fig. 4b is a schematic illustration of a surface treatment corresponding to the second stage of the method for forming retro-reflective devices according to the present invention.
- Fig. 5 is a schematic illustration of a rolling process followed by a surface treatment process according to the present invention.

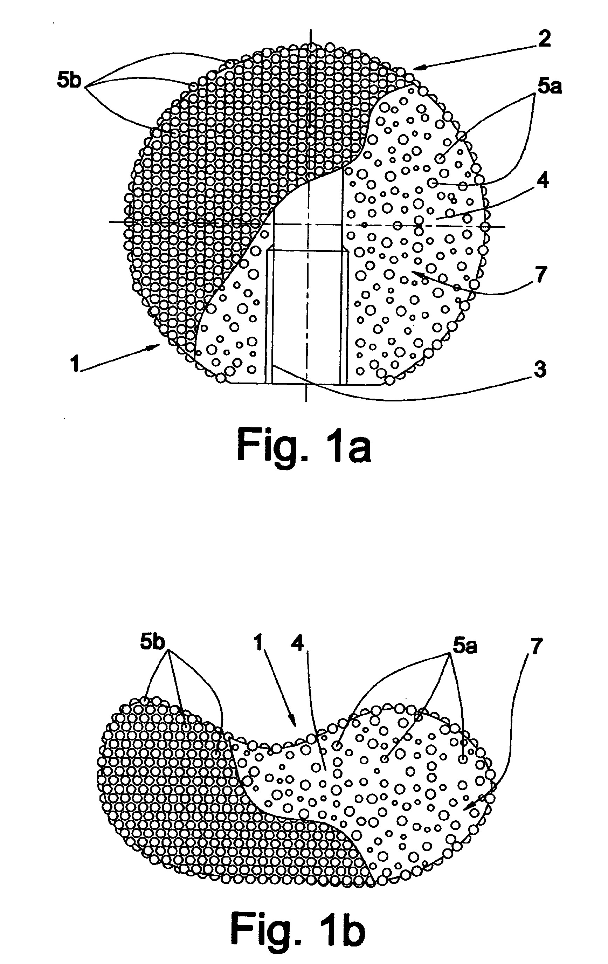

Fig. 1a and 1b show examples of retro-reflective devices 1 according to the present invention. Fig. 1a shows an essentially spherical device, which could be typically designed to be used as a passive marker in IGS systems, whereas Fig. 1b shows a more complicated tri-dimensional device having a concave portion on its top part, as shown here in a partial top sectional view.

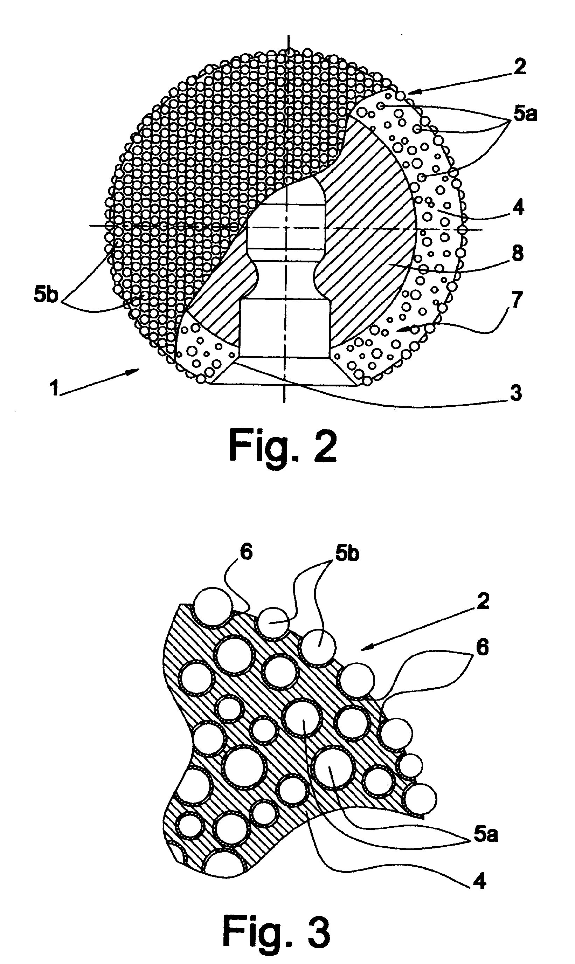

Referring to Fig. 1a, the retro-reflective device 1 consists of at least a body 2, more particularly a spherical body, which for example can also include one latching means 3 allowing to secure it on a tool or any other support for its use. As shown in this figure, such a latching means 3 can be a thread for instance or any other internal securing means. As a variant, such a latching means could also consist of an extending fastening means, protruding out of the body 2. A partial sectional view is shown on the right part of Fig. 1a. This view shows that the body 2 is made of at least one first material entity 7 made of a homogeneous compound of two main constitutive elements, namely plastic material 4 and elements 5a, 5b, here illustrated by micro-spherical elements. Elements 5a are fully lodged inside the plastic material 4, whereas the elements 5b emerge from the outer surface of this plastic material as better shown in Fig. 3.

Fig 1b shows a more complicated tri-dimensional device having a concave portion on its top part. On this figure, also shown here in a partial sectional view, it appears that the retro-reflective device 1 of the present invention can be made from any kind of shapes.

Fig. 2 is a partial sectional view of another essentially spherical retro-reflective device 1, made of a first material entity 7 and at least one additional material entity 8 without any elements 5a, 5b. Such an additional material entity 8 is a distinctive part constituting the body 2. This part is substantially distinguishable from the first material entity 7 by its type, namely its material and/or its shape. Such a retro-reflective device can be realized by a bi-compound injection machine. Bi-compound injection makes it possible to produce devices combining several functions by optimum use of the properties of the material involved.

As illustrated in a preferred embodiment, this additional material entity 8 forms the core of the body 2. Preferably, this core is made of a material whose thermal expansion coefficient is substantially higher than that of the compound constituting the first material entity 7, and more specifically higher than the thermal expansion coefficient of the plastic material 4. Owing to such different physical properties, a steam sterilization or any other sterilization at high temperature of the retro-reflective device will produce its destruction, particularly the destruction of the first material entity 7 which covers the core of the body 2. Such a destruction should be considered as being an advantage on the security and reliability aspect, in particular when any incident is strictly prohibited and must always be avoided, such as during a chirurgical operation.

Preferably, the aforementioned additional material entity 8 is made of a resilient material. Such a resilient property allows to realize a user-friendly snap-in function to the surgical tool. For example, Fig. 2 also shows an internal latching means 3 expected to secure the retro-reflective device 1 on a non illustrated specific support, simply by clipping it.

Preferably, the additional material entity 8 is made of a resilient material, such as an elastomer, and/or is shaped to have a flexible property. Preferably, this additional material entity 8 is made of injectable polymer such as thermoplastics having also a certain malleability. Several kinds of injectable polymers will be given hereafter with reference to the description of Fig. 3.

Fig. 3 is an enlarged sectional view of a portion of the body 2, and more specifically of a portion of the first material entity 7 which constitutes this body 2. On this drawing, it is shown that the elements 5a, which are fully embedded inside the plastic material 4, are entirely coated with at least one reflective layer 6, whereas the salient non-coated surface of elements 5b, protruding out of the surface of the plastic material 4, are partially coated with the same reflective layer 6. The external surface of the salient elements 5b are removed from the reflective layer 6, whereas the internal surface of these salient elements 5b are still coated with such a reflective layer 6. Owing to this feature, salient elements 5b become retro-reflective elements in the case where the device 1 is put under an external light source. Preferably, salient elements protrude out of the surface of the plastic material up to 60% of their diameter.

Elements 5a, 5b, have the shape of lenses intended to be used for their optical properties. Preferably, they are sphere shaped and most preferably are made of transparent micro-spheres or beads whose diameter is in the range of 10 to 5000 micrometers. These micro-spheres are made of a material which has a refractive index comprised between 1.5 and 2.9. For example, such a material can be glass or transparent non-vitreous material.

The reflective material of the layer 6 which is applied on the elements can be made of aluminium, silver or any other reflective material well adapted to the used wavelength. Such a material can be applied on the entire surface of the elements by a conventional physical vapor deposition process (PVD) for example. This PVD coating process guaranty the best contact between the element and the reflective material and therefore the best reflectivity coefficient achievable for a given coating material. The thickness of the reflective layer surrounding the elements measures at least 50nm, preferably from 50nm up to 1000nm in order to get a high reflectivity. Silver can be used for this coating layer due to the fact that it has an excellent reflectivity in the infrared domain and shows a bactericide property which is of great interest for the medical domain. Aluminium could be also used owing to its lower cost and its good hardness characteristic.

The plastic material 4 is made of injectable polymer or any other polymer having a certain malleability. It can be transparent as well as opaque. Such a plastic material could be for example polycarbonate (PC), polymethyl methacrylate (PMMA), polystyren (PS), polyethylen (PE), polyurethan (PUR), polyvinyl chloride (PVC) or any other thermoplastic.

The mixture or compound which constitutes the first material entity 7 and which comprises elements 5a, 5b and plastic material 4, is made as homogeneous as possible. Moreover, to get a maximum of elements 5b at the surface of the body 2, the elements 5a, 5b into the compound are maximized. It is known that the theoretical limit of concentration for spherical elements of same diameter gives an occupation of 74% of the entire volume (face centered cubic arrangement). It is also known that a random repartition of identical spheres gives a maximum concentration of 64%. To get an elements concentration up to 90%, the present invention suggests to use a compound including elements of several different sizes. A solution consists of mixing the plastic material with beads having two or more different diameters. This solution allows to the higher diameter beads to roll over the smaller beads and allows the latter to slip between the bigger ones. By this way, the concentration rate of beads in the plastic material can be increased while keeping the injectability of the compound. Another solution consists in having a continuous variation of the sizes or diameters of the elements 5a, 5b, so as to still increase the concentration rate. Therefore, the diameters of transparent micro-spheres can vary in a continuous manner between the smallest diameters and the biggest ones. Owing to these features, the elements concentration can be comprised between 40-90% of the entire volume of the first material entity 7.

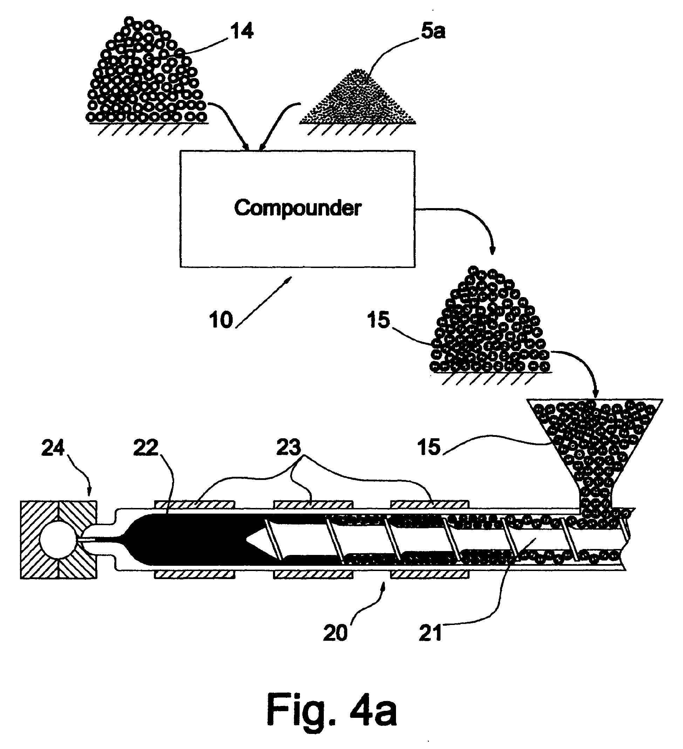

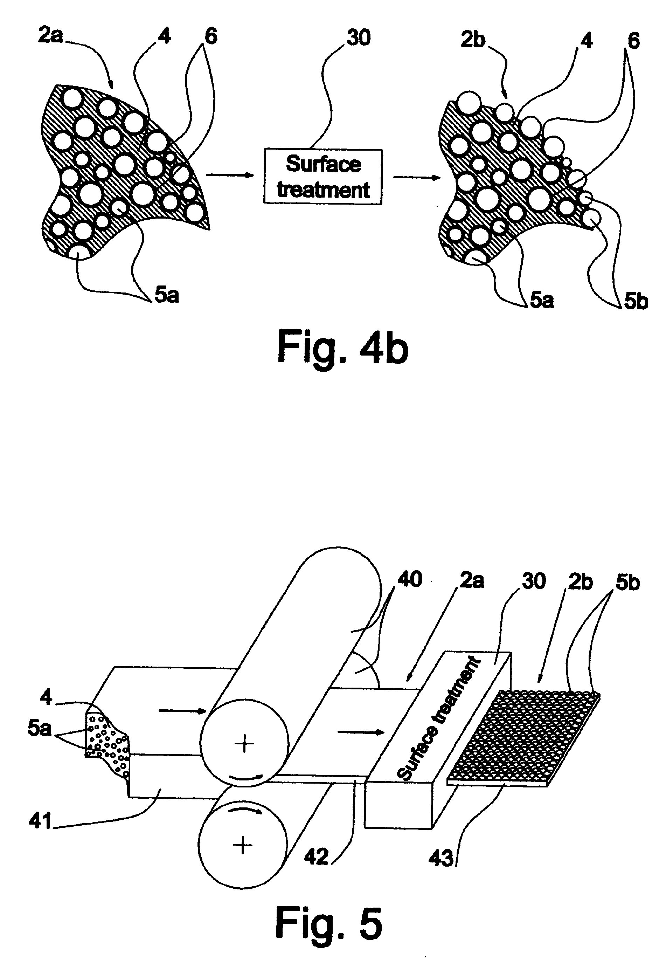

Referring now to Fig. 4a, it shows a schematic illustration of a converting process, more particularly an injection process, which corresponds to the first stage of the method for forming retro-reflective devices as described here-above. The second stage of this method will be explained later with the help of schematic illustration given in Fig. 4b.

The aim of the first stage of this method is to obtain at least a compound made of plastic material 4 and entirely coated elements 5a with at least one reflective layer 6, so as to obtain a homogeneous first material entity 7 of a primary body 2a which will be described hereafter with the second stage of the method. To this end, it is first suitable to mix, in a conventional compounder 10, elements 5a with pellets 14 made of plastic material 4. The amount of elements with respect to the amount of pellets 14 are in the range of the rate concentration previously disclosed. In the same manner, the characteristics of elements 5a and of plastic material 4 also correspond to those previously disclosed in connection to the description of the retro-reflective device 1.

As shown in Fig. 4a, the compounder 10 is fed with the plastic material pellets 14 and the elements 5a. Cold mixing of pellets 14 and elements 5a is then heated. Owing to the increasing of the temperature, the plastic material pellets melt until to form a homogeneous paste including elements 5a. This paste is then ejected by a nozzle and hashed so as to form charged pellets 15 including the elements 5a.

Further to this first transformation, the charged pellets 15 are then fed into a converting machine 20 such as an injection molding machine. Other converting machines not represented in Fig. 4a can be used, in place of the molding machine, such as machines for extrusion, blow molding, blow extrusion, cast molding, rolling or any other one compatible with the used plastic material 4. Therefore, it should be kept in mind that "converting machine" wording refers to any kind of machine allowing to convert charged pellets 15 so as to obtain a new material shaped by that converting machine.

The converting machine 20 shown in Fig. 4 refers to a conventional injection molding machine. It comprises a screw 21 for conveying the charged pellets 15 toward the outlet of the converting machine 20. In order to gradually melt the charged pellets 15 until to obtain a charged paste 22, heaters 23 are also settled along the screw 21. At the outlet of the converting machine 20, the charged paste 22 is finally injected into a mold 24, whose the inside part corresponds to the desired shape of the body 2 of the retro-reflective device 1. It is clear that the injection molding machine illustrated in Fig 4a can also be provided with multi cavity mold in order to increase the productivity of this process.

After several workshop tests, it appears that the first transformation makes it possible to avoid jamming of the injection molding machine by elements 5a and also makes it possible to obtain a more homogeneous charged paste 22.

The second stage of the method refers to Fig. 4b which is a schematic illustration of a surface treatment 30 applied onto the body once it is taken out of its mold 24. In order to distinguish the body 2 before and after this external treatment, the body as picked up from its mold will be named the primary body 2a, and after this treatment will be named the treated body 2b. As shown in Fig. 4, the external surface of the primary body 2a is fairly smooth and no beads protrude out of this surface. On the contrary, the external surface of the treated body 2b includes elements 5b emerging out of the surface of the plastic material 4.

The first action of the surface treatment 30 consists of removing an external layer of plastic material 4 of the primary body 2a until the coated elements, held close to the surface of the primary body, protrude out of this surface up to 60% of their diameter. The second action of the surface treatment 30 consists of removing the reflective layer 6 emerging out of the surface of the body for each element protruding out of this surface. To this aim, surface treatment could be obtained as different manners with several various means such as brushing, chemical attack with a polymer solvent and/or a coating solvent, thermal treatment, photo ablation, sand blasting, vibro-finishing or burr removal. If necessary, some of these means can be followed by a final polishing stage, with or without solvent.

Several of those means can also be combined so as to obtain a better outcome. Those means, or combination of means, can either be used for removing both the external layer of plastic material 4 and the reflective layer 6 in one step, or can be used to first remove the appropriate external layer of plastic material 4, for example with one means, and then to use a second means to remove the reflective layer 6. Moreover, those means, or a combination of these means, can be used either simultaneously or successively in order to achieve the removal of the external layer of plastic material 4 and of the reflective layer 6. Removing of those layers can be also achieved by one same means.

The brushing means is preferred because it corresponds to a smooth means wherein a solvent can be easily added in order to increase the removing effect of the plastic material 4 or of the reflective layer 6 covering the external elements 5b. Owing to the flexibility of the brush bristles of this means, these bristles are deflected by the elements 5b so that a meniscus of polymer always stay at the base of the elements making a good anchorage. The deflecting of the bristles around the elements also favor their freeing during the surface treatment.

Although being manually achievable, the second stage, corresponding to the surface treatment, of the described method will be preferably an automated treatment.

Regarding to the plastic material 4, namely the material of the pellets 14, it is made of an injectable polymer, or any other polymer having a certain malleability, whose features are first a very good adhesion with the elements and second a good consistency when heated. Therefore, the heated plastic material 4 must have on the one hand a sufficient fluidity to be easily injected into molds, and on the other hand a sufficient viscosity so as to keep homogeneous the charged paste 22. To this aim, the aforementioned polymers disclosed in connection with the description of the retro-reflective device 1 are well suitable.

A great advantage induced by this new manufacturing technology is that the mechanical precision of the retro-reflective device 1 is given by the precision of the mold 24. This property is very important for IGS systems where the precision of the calculated position of a surgical tool is linked directly with the precision of the passive marker dimensions. Therefore, the subject of the present invention also refers to a preferred use of retro-reflective devices 1 to synthesis images applications such as IGS systems or computer-generated imagery used in cinema for example.

Referring now to Fig. 5, it shows a schematic illustration of a rolling process followed by the surface treatment 30 previously mentioned. The purpose of the Fig. 5 is to show an other application of the present invention. A compound 41 made of plastic material 4 and entirely coated elements 5a with a reflective layer 6 is used to fed a rolling 40. At the outlet of this converting machine comes out a flat-rolled product 42. The flat-rolled product 42 has a primary surface 2a with a smooth finish and no elements protrude out of the surface. The surface treatment 30 is then applied over the surface 2a and the final treated product 43 acquires the retro-reflective property over the treated surface 2b. This process can also be used to get a multi-layer laminated product 43 by feeding the rolling machine with a multi layer compound 41.

As shown in Fig. 1a, Fig. 1b, Fig. 2 and Fig. 5, the device of the present invention can look in any shape such as voluminous or thin articles, having regular or irregular external surfaces, with or without concave parts for instance. Additionally, the surface treatment 30 can be applied only partially to the body 2a in a way to obtain reflective zones and non reflective zones. This could be used for instance to write symbols or letters over panels like road signs.

## Claims

1. Retro-reflective device (1) having at least a body (2) comprising salient elements (5b) on an external surface of this body, **characterized by** the fact that said body is made of at least one first material entity (7) made of a homogeneous compound of plastic material (4) with on one hand elements (5a) entirely coated with at least one reflective layer (6) and fully embedded inside the plastic material (4), and on the other hand salient elements (5b) partially coated with the same reflective layer (6), said reflective layer located inside the plastic material (4), and the salient non-coated surface of said elements (5b) protruding out of the surface of the plastic material (4).

2. Retro-reflective device (1) according to claim 1, **characterized by** the fact that the body (2) is made of at least one additional material entity (8) without any elements (5a, 5b).

3. Retro-reflective device (1) according to claim 2, **characterized by** the fact that said additional material entity (8) forms a core having a thermal expansion coefficient which is substantially higher than the thermal expansion coefficient of the plastic material (4).

4. Retro-reflective device (1) according to claim 2, **characterized by** the fact that said additional material entity (8) is made of a resilient material and/or is shaped to have a flexible property.

5. Retro-reflective device (1) according to claim 2, **characterized by** the fact that said plastic material (4) and said additional material entity (8) are made of injectable and/or malleable polymer.

6. Retro-reflective device (1) according to claim 1, **characterized by** the fact that said compound is made of elements (5a, 5b) concentration in the range of 40-90% of the entire volume of the first material entity (7).

7. Retro-reflective device (1) according to claim 1, **characterized by** the fact that salient elements (5b) protrude out of the surface of the plastic material (4) up to 60% of their diameter.

8. Retro-reflective device (1) according to claim 1, **characterized by** the fact that elements (5a, 5b) are made of transparent micro-spheres, said transparent micro-spheres are made of glass material, have diameters comprised between 10 and 5000 micrometers, have a refractive index comprised between 1.5 and 2.9 and have a reflective layer (6) thickness of at least 50nm.

9. Retro-reflective device (1) according to claim 8, **characterized by** the fact that the diameters of transparent micro-spheres vary in a continuous manner between the smallest diameters and the biggest ones.

10. Method for forming retro-reflective devices (1), said method comprising, for forming each retro-reflective device (1):
a) converting process of at least a compound containing plastic material (4) and elements (5a), entirely coated with at least one reflective layer (6), so as to obtain a homogeneous first material entity (7) of a primary body (2a);
b) achieving of a surface treatment (30) of said primary body (2a) in order to render salient elements held close to the surface of the primary body (2a) and to remove the reflective layer (6) protruding out of the surface of this body for each salient elements (5b).

11. Method according to claim 10, wherein the converting process of the compound containing plastic material (4) and elements (5a) comprises the following steps:
a1) mixing of pellets (14), made of plastic material (4), with elements (5a), entirely coated with a reflective layer (6), until achieving a homogeneous compound;
a2) hashing of said compound so as to obtain charged pellets (15) made of plastic material (4) including said coated elements (5a), then
a3) feeding of said charged pellets (15) into an converting machine (20, 40) so as to obtain a primary body (2a) shaped by said converting machine (20, 40).

12. Method according to claim 10, wherein the surface treatment (30) of the primary body (2a) comprises the following actions:
b1) removing an external layer of plastic material (4) of said primary body (2a) until the coated elements (5a), held close to the surface of the body, protrude out of this surface up to 60% of their diameter;
b2) removing the reflective layer (6) emerging out of the surface of the body (2).

13. Method according to claim 12, wherein removing said external layer of plastic material (4) and removing said reflective layer (6) emerging out of the surface of the body (2) are achieved by one same means or a combination of different means, either simultaneously or successively.

14. Method according to claim 12, wherein removing said external layer of plastic material (4) is achieved by a first means and removing said reflective layer (6) emerging out of the surface of the body (2) is achieved by a second means different from the first means.

15. Method according to claim 13 or 14, wherein said means are either brushing means with or without solvent, or chemical attack means, or thermal treatment means, or photo ablation means, or sand blasting means, or vibro-finishing means, or polishing means, or burr removal.

16. Use of the retro-reflective device (1) according to any of claims 1 to 9 to synthesis images applications.
